# EUROPEAN PATENT SPECIFICATION

(11) **EP 2 643 503 B1**
(45) Date of publication and mention of the grant of the patent: **05.11.2014**
(21) Application number: 11802296.1
(22) Date of filing: 23.11.2011
(51) Int. Cl.: D01D 5/00, D01F 1/10, D01D 5/38, A61L 27/14, A61L 27/38, A61L 17/00

(54) **PRODUCT OF CROSSLINKED MATERIAL AND METHOD FOR PRODUCING THE SAME**
PRODUKT AUS VERNETZTEM MATERIAL UND VERFAHREN ZU SEINER HERSTELLUNG
PRODUIT DE MATÉRIAU RÉTICULÉ ET PROCÉDÉ DE FABRICATION ASSOCIÉ

(30) Priority: 24.11.2010 EP 10192395
(43) Date of publication of application: 02.10.2013
(73) Proprietor: SpinPlant GmbH, 04109 Leipzig (DE)
(72) Inventor: MEISEL, Jörg, Berlin 14163 (DE); GANEY, Timothy, Tampa Florida 33604 (US)
(74) Representative: Sokolowski, Fabian
(86) International application number: PCT/EP2011/070871
(87) International publication number: WO 2012/069559

(56) References cited:
- WO-A2-2008/082588
- WO-A2-2009/011944
- WO-A2-2009/064767
- US-A1- 2010 183 699
- US-B2- 6 565 960

## Description

The invention relates to a method for producing a nanofiber-based product according to the preamble of claim 1, a product which can be obtained by such a method according to the preamble of claim 12 and the use of such a product according to claim 15.

It is known from prior art to electrospin different polymers into nanofibers. However, the stability of these nanofibers is often not satisfactory high. When the electrospun fibers are brought into contact with water or another solvent, dissolution of the fibers often occurs. Many approaches to stabilize the polymers by crosslinking have failed.

It is an object of the invention to provide a method by which highly stable nanofiber-based products can be obtained as well as to provide such nanofiber-based products.

This object is achieved by a method having the characteristics of claim 1. This method for producing a nanofiber-based product comprises the steps of providing a carrier material solution which itself comprises a carrier material, and bringing the carrier material in contact with the collector by electrospinning the carrier material solution out of the spinning device. Thereby, the collector has a first electrical polarity and the spinning device has a second electrical polarity which is opposite to the first polarity. According to the invention, the carrier material essentially consists of a polymer which is - at least after it has contacted the collector - chemically crosslinked with the residue of a crosslinker and/or embedded in a polymer formed by the crosslinker. The carrier material is preferably finally crosslinked and/or finally embedded already immediately after having contacted the collector, i.e. upon contact with the collector. In other words, the carrier material is solved or dispersed together with the crosslinker in the carrier material solution and is then precipitated from the carrier material solution under electric field strength onto the collector. Due to this precipitation, the crosslinking and/or embedding reaction ends. Alternatively, the crosslinker is added to the carrier material solution during electrospinning. Also in this case, crosslinking is terminated upon carrier material precipitation on the collector.

It turned out that electrospun fibers which had been crosslinked after electrospinning did not show an enhanced stability with respect to non-crosslinked nanofibers. Therefore, it is crucial that the crosslinking is finished upon formation of the nanofibers. This is presently the case when the carrier material contacts the collector, because carrier material and crosslinker are electrospun together.

It has been also turned out that not any generally known crosslinker can be used to successfully crosslink the polymer, but only a crosslinker which is a compound according to the general formula (I) 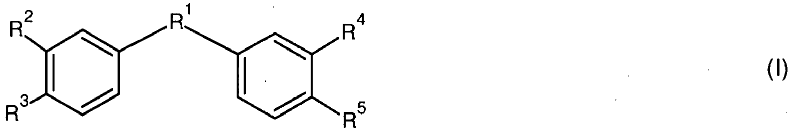 wherein R¹ is
- a single bond between the adjacent carbon atoms, or
- a carbohydrate chain having 1 to 10 carbon atoms and optionally bearing a hydroxy group, the carbohydrate chain being saturated, unsaturated or polyunsaturated,
and wherein R², R³, R⁴ and R⁵ are independently from each other
- a hydrogen,
- a carbohydrate chain having 1 to 10 carbon atoms and optionally bearing a hydroxy group, the carbohydrate chain being saturated, unsaturated or polyunsaturated,
- a hydroxy group, or
- a sulfhydryl group,
with the provision that the compound bears at least
- two hydroxy groups, or
- two sulfhydryl groups, or
- one hydroxy group and one sulfhydryl group.

The term "crosslinker" is used in the entire text to encompass chemical substances which crosslink the carrier material by forming chemical bonds to the carrier material and/or by forming a polymer in which the carrier material is embedded. It is possible that chemical bond formation between crosslinker and carrier material also takes place in case of a polymer of crosslinker in which the carrier material is embedded. Thus, the carrier material can intercalate a polymer of a crosslinker and additionally can form chemical bonds to the crosslinker. Alternatively, only chemical bond formation (without polymeric embedding or intercalation) can take place. In a further embodiment, only polymeric embedding (without chemical crosslinking) is effected. A substance comprising a polymer of a crosslinker and an embedded (or embedded and at least partially chemical linked) carrier material can also be denoted as composite or as nanofiber reinforced composite.

During electrospinning, a voltage difference between the electrified carrier material to be spun and the grounded collector exists. This voltage difference ameliorates crosslinking processes of the single polymer molecules of the carrier material when a crosslinker belonging to the above-explained group is used.

Well-suited examples for photoreactive diazines are photoreactive analogues to leucine and methionine.

In an embodiment R¹ is a residue having the following formula (II) 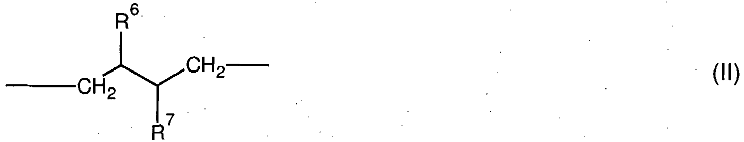 wherein each of the two terminal methyl residues (indicated as CH₂) is linked to a carbon atom of each benzene ring (the possible link is indicated by single bonds that are not connected to any atom in formula (II)) and wherein R⁶ and Fez are independently from each other
- a hydrogen,
- a carbohydrate chain having 1 to 10 carbon atoms and optionally bearing a hydroxy group, the carbohydrate chain being saturated, unsaturated or polyunsaturated,
- a hydroxy group, or
- a sulfhydryl group.

This means that in this embodiment the two benzene rings of the compound according to formula (I) are linked together by an optionally derivatized butyl chain, leading to a compound of the general formula (III):

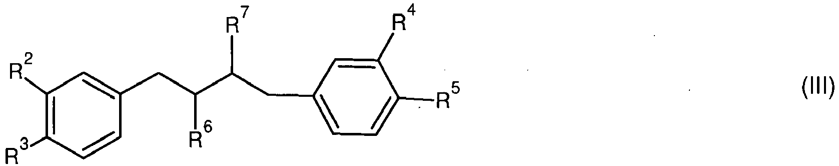

In an embodiment, at least two hydroxy groups or at least two sulfhydryl groups or at least one hydroxy group and one sulfhydryl group of the compound according to the general formula (I) are bound to a single or to both benzene rings of this compound. By binding the hydroxy and/or sulfhydryl groups to the benzene rings, a higher reactivity of the hydroxy and/or sulfhydryl group is achieved which leads to a better crosslinking performance of the according compound. The reactive groups (hydroxy and/or sulfhydryl) can be positioned in the benzene rings in ortho, meta or para position to each other, wherein an ortho position is preferred.

In a further embodiment, the crosslinker bears at least one, preferably at least two, preferably exactly two catechol groups. Thus, the crosslinker might be a di-catechol or an according derivative.

In a further embodiment, all hydroxy and/or sulfhydryl groups of the compound are bound to the benzene rings and no further such groups exist in the molecule.

The compound according to the general formula (I) mediates crosslinking of polymers particularly by its hydroxy or sulfhydryl groups. For example, an ether bridge can be formed by reaction of a hydroxy group of the compound according to the general formula (I) and the polymer. Alternatively, a disulfide bridge may be formed between the sulfhydryl group of the compound according to the general formula (I) and a sulfhydryl group of a polymer to be crosslinked. Since the compound according to the general formula (I) bears at least two reactive groups (hydroxy and/or sulfhydryl), it has at least two reactive centers so that long cross-linked networks can be produced. The more hydroxy and/or sulfhydryl groups the compound according to the general formula (I) bears, the higher branched can be the resulting crosslinked polymer.

The crosslinker might also react with only specific amino acids of the carrier material to be crosslinked if this carrier material bears amino acids. For example, the crosslinker might only react with lysine or arginine residues of the carrier material but not with other amino acid residues of the carrier material. The stiffer material properties following crosslinking with a crosslinker, in particular with NDGA, suggest that similar chemistries that are shown in cartilage may take place as well. It was previously described that advanced glycation end products of arginine and lysine contribute to stiffness, color, and an apparent reduction in both amino acids noted above. In a preferred embodiment, the crosslinker reacts with specific amino acids of the carrier material and additionally forms a polymer in which the carrier material can be embedded.

In an embodiment, the crosslinker is not any of the following compounds: multifunctional aldehydes (e.g., glutaraldehyde), multifunctional acrylates (e.g., butanediol diacrylate), halohydrins (e.g., epichlorohydrin), dihalides (e.g., dibromopropane), disulfonate esters, multifunctional epoxies (e.g., ethylene glycol diglycidyl ether), multifunctional esters (e.g., dimethyl adipate), multifunctional acid halides (e.g., oxalyl chloride), multifunctional carboxylic acids (e.g., succinic acid), carboxylic acid anhydrides (e.g., succinic anhydride), organic titanates (e.g., TYZOR from DuPont), dibromoalkanes, melamine resins (e.g., CYMEL 301, CYMEL 303, CYMEL 370, and CYMEL 373 from Cytec Industries, Wayne, N. J.), hydroxymethyl ureas (e.g., N,N'-dihydroxymethyl-4,5-dihydroxyethyleneurea), multifunctional isocyanates (e.g., toluene diisocyanate or methylene diisocyanate).

A possible polymerization scheme for polymer formation of the crosslinker is explained in the following. Assuming the crosslinker being a di-catechol derivative, 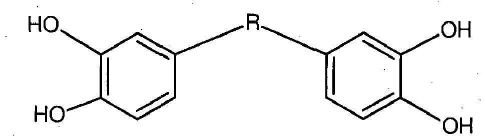 oxidation to a di-quinone takes place:

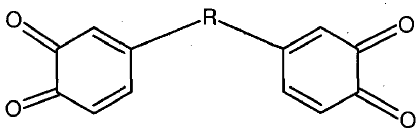

Two di-quinones react then via aryloxy free radical generation and subsequent oxidative coupling as well as reoxidation to a bisquinone:

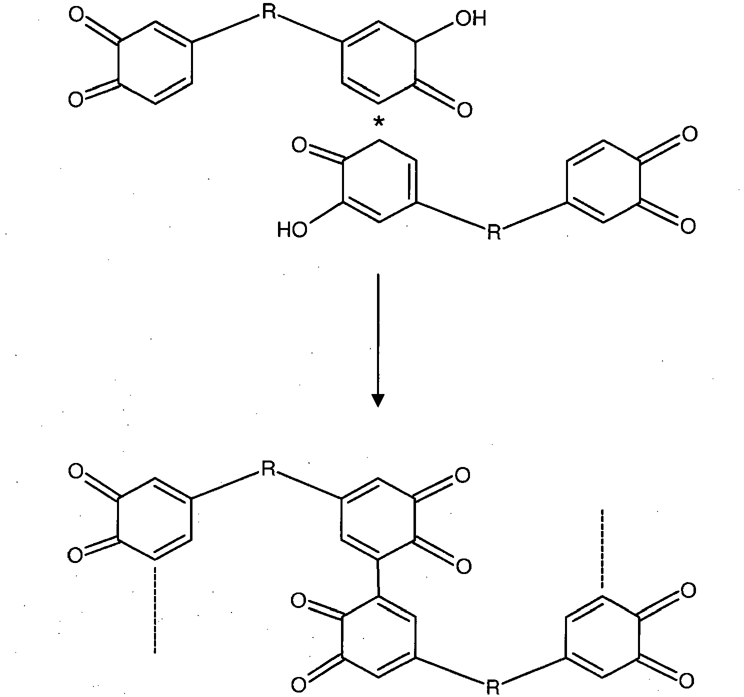

Further equivalent reactions lead to an extended polymer of bisquinone units so that a bisquinone polymer is formed. The carrier material can well intercalate this bisquinone polymer and can thus be embedded in it. A re-reduction of the carbonyl groups to hydroxyl groups in the polymer is possible.

In an embodiment, the compound according to the general formula (I) bears more than three, in particular more than four, in particular more than five, in particular more than six, in particular exactly four reactive groups of the type hydroxy and/or sulfhydryl.

In a further embodiment, the crosslinker is nordihydroguaiaretic acid (NDGA). The structure of NDGA is the following:

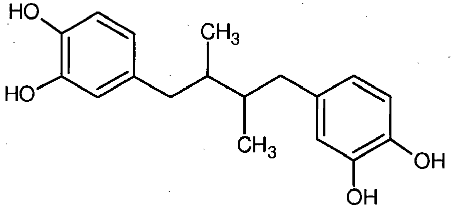

It turned out that NDGA is a very well suited crosslinker for crosslinking the polymer of the carrier material. Nanofibers of the carrier material being crosslinked by NDGA show a very high stability and are very long-lasting in solutions in which non-crosslinked polymers already dissolve. NDGA forms a polymer into which the carrier material is embedded.

In a further embodiment, the crosslinker is used in an amount of 2 to 20 wt % with respect to the dry mass of the polymer of the carrier material. Further suited amounts are 5 to 15 wt %, in particular 7 to 13 wt %, in particular 9 to 11 wt %, in particular around 10 wt %.

In a further embodiment, the carrier material is crosslinked before it reaches the collector. In doing so, cross-linking can take place particularly well not only on the surface of the nanofibers already being electrospun, but also internally. This enhances the stability of the nanofibers themselves and between each other. As explained above, it is crucial that the crosslinking is finished upon formation of the nanofibers. This is the case when the carrier material contacts the collector. If crosslinking is already finished a certain while before the carrier material contacts the collector, the integrity of the nanofibers is even more enhanced when the impact of the contact with the collector acts upon them.

In a further embodiment, the carrier material comprises of collagen, a mixture of collagen and hydroxy apatite, gelatin, alginates, chitosan, silk, cellulose, polyurethane, a polyester, polycaprolactone, polylactide, polypyrrole, polyaniline, polyacetylene, polythiophene, a copolymer of the preceding polymers and/or a copolymer bearing carboxylic acid groups and/or amine groups.

Further suited carrier materials are amino acid structures like oligopeptides or polypeptides. Oligopeptides are considered to consist of a sequence of up to 10 amino acids, whereas polypeptides are considered to be amino acid structures having more than 10 amino acids. Proteins are to be considered to be encompassed by the term "polypeptide". This means the amino acids structure can exhibit a primary, secondary and tertiary structure by itself so that the structure of the material to be deposited on the collector can exhibit an individually adjusted sub-structure. By using an amino acid structure, one can take advantage not only of the secondary or tertiary structure of the oligopeptides or polypeptides, but also of the amphiphilic charge of the single amino acids being present in the oligopeptides or polypeptides.

Well-suited collagens are collagen type I, II, III, V, or XI, wherein type I collagen is particularly well suited. The collagen might for example have a human, bovine, equine, ovine or fish origin or can be an artificial collagen resembling human, bovine, equine, ovine or fish collagen, or might consist of collagen, or collagen fibrils that have been expressed in culture by vector incorporation without limitation to mammalian source.

A particularly well-suited material is collagen. Using collagen as carrier material and NDGA as crosslinker leads to very well crosslinked and stable collagen nanofibers. They can be used in a particularly advantageous manner for producing a product according to the instant method.

The spinning device may be a nozzle through which the carrier material solution can be sprayed, or a turning (or rotating) pin located in a bath of the carrier material solution. By turning or rotating the pin in the bath, the carrier material solution is transported out of the bath and accelerated in the direction of the collector.

In an embodiment, the electrospinning may be performed at a voltage of 8 to 20 kV, in particular of 10 to 17 kV, in particular of 12 to 15 kV between the collector and the spinning device. A voltage of approximately 12.5 kV is particularly suited. Those voltages are well suited for accelerating the carrier material solution sufficiently fast out of the spinning device towards the collector. Further electrospinning parameters like the speed of a rotating pin for ejecting some of the carrier material solution out of a reservoir of the carrier material solution or the flow rate of the carrier material solution towards a nozzle, and the distance between the spinning device and the collector can be adjusted to the respective needs according to standard protocols of electrospinning.

In a further embodiment, the polymer is solved or dispersed in at least one liquid chosen from the group of water, alcohols like methanol or ethanol, aqueous solutions of acids or bases like acetic acid or sodium hydroxide and organic solvents like acetone or 1,1,1,3,3,3-hexaflouoro-2-propanol to produce a carrier material solution.

The term "carrier material solution" also encompasses "carrier material dispersions". This means, it is not necessary that the carrier material is ideally solved in an according liquid. If an essentially stable dispersion of the carrier material in an according liquid is established, electrospinning can also take place.

In a further embodiment, the carrier material is deposited onto the collector to form a sheet material. Such a sheet material can be used as bone substitute material or as wound-covering fleece.

In an alternative embodiment, the carrier material is removed from the collector after having contacted it and is then spun to a yarn. Such a yarn may be used as suture material in orthopaedic or surgical applications. The linear material, or yarn, or bioyarn, may also be embodied as a raw material characterized by its strength, stiffness, elasticity, modulus, charge and composition as to make it a linear material that can be braided, woven, knitted, plied, or in other ways converted to mesh, fabric, matted material, or laminated with specific endowment as to afford areas between the threads to dynamic as well as static properties that align to the degradation of the material and regeneration of the biologic tissue.

In a further embodiment, the electrospun carrier material is washed after having been removed from the collector. By washing, remainders of non-reacted crosslinker or reaction co-products can be removed, thus improving the biocompatibility of the obtained product. Washing can be performed by an aqueous solution of an alcohol like for example ethanol in a concentration of for example 80 %, 70 %, 60 % or 50 % (v/v). Further suited washing solutions are low salt buffers like phosphate buffered saline (PBS). Washing can be performed in a two-step manner by first using an alcoholic solution and afterwards a low salt buffer (or first a low salt buffer and afterwards an alcoholic solution) as washing solutions.

In an alternative embodiment, no washing step is necessary due to the specific crosslinker chosen and the concentration in which it is used.

The object is also achieved by a product which can in particular be obtained by a method according to the preceding explanations. Such product comprises a carrier material being built up from nanofibers having a diameter of less than 1 200 nm, in particular less than 1 100 nm, in particular less than 1 000 nm, in particular less than 900 nm, in particular less than 800 nm, in particular less than 700 nm, in particular less than 600 nm, in particular less than 500 nm, in particular less than 400 nm, in particular less than 300 nm.

According to the invention, the carrier material essentially consists of a polymer which is chemically crosslinked with the residue of a crosslinker. This crosslinker is a compound according to the general formula (I) 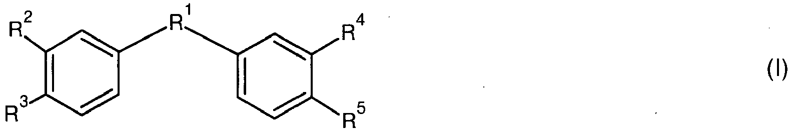 wherein R¹ is
- a single bond between the adjacent carbon atoms, or
- a carbohydrate chain having 1 to 10 carbon atoms and optionally bearing a hydroxy group, the carbohydrate chain being saturated, unsaturated or polyunsaturated,
and wherein R², R³, R⁴ and R⁵ are independently from each other
- a hydrogen,
- a carbohydrate chain having 1 to 10 carbon atoms and optionally bearing a hydroxy group, the carbohydrate chain being saturated, unsaturated or polyunsaturated,
- a hydroxy group, or
- a sulfhydryl group,
with the provision that the compound bears at least
- two hydroxy groups, or
- two sulfhydryl groups, or
- one hydroxy group and one sulfhydryl group.

In an embodiment, the molecular ratio (i.e. the number of molecules of the first species divided by the number of molecules of the second species) between the monomeric units of the carrier material and each residue (i.e. each monomeric unit of a formed polymer) of the crosslinker is in the range of 20:1 to 5:1, in particular of 15:1 to 7:1, in particular of 12:1 to 8:1, in particular of 11:1 to 9:1 in the product.

In an embodiment, the product further comprises at least one auxiliary substance of the group consisting of osteoinductive substances, electrically conductive substances, electrically semiconductive substances, electrically insulating substances, antibacterial substances, antiviral substances, antifungal substances, ceramics (like, e.g., hydroxyapatite ceramics), barium, copper, bromine, niobium, lithium, germanium, titanium, lead, zirconium, silicon, silver, zinc, polyurethane and silver hydrogen sulfate, gallium orthophosphate (GaPO₄), langasite (La₃Ga₅SiO₁₄), barium titanate (BaTiO₃) lead titanate (PbTiO₃), lead zirconate titanate (Pb[ZrₓTi₁₋ₓ]O₃ 0<x<1), potassium niobate (KNbO₃), lithium niobate (LiNbO₃), lithium tantalate (LiTaO₃), sodium tungstate (Na₂WO₃), Ba₂NaNb₅O₅ and Pb₂KNb₅O₁₅, wherein the auxiliary substance is chemically and/or physically bound to the carrier material.

By such an auxiliary substance, additional biological and/or chemical effects can be introduced into the product. The auxiliary substance can be entrapped within the polymer network of the carrier material or the carrier material and the crosslinker, respectively. The entrapment offers the effect that a certain placement/precipitation of the auxiliary substance can be achieved, e.g. a placement/precipitation in the banding structure of the carrier material perpendicular to scaffold orientation. Thus, if hydroxyapatite is used as auxiliary substance and collagen is used as carrier material, the natural orientation of hydroxyapatite with respect to collagen in normal bone (i.e. in the banding structure of bone perpendicular to scaffold orientation) can be mimicked.

In an embodiment, the product exhibits an maximum tensile strength of ca. 50 to 200 MPa, in particular of ca. 75 to 175 MPa, in particular of ca. 90 to 150 MPa, in particular of ca. 100 to 125 MPa.

In a further embodiment, the product exhibits an elastic modulus of ca. 300 to 700 MPa, in particular of ca. 350 to 650 MPa, in particular of ca. 400 to 600 MPa, in particular of ca. 450 to 580 MPa, in particular of ca. 500 to 550 MPa.

Further embodiments explained with respect to the claimed method can also be applied in an analogous way to the claimed product and are not repeated here for the sake of brevity only.

A product having the characteristics as explained above can be very well used as scaffold for growing cells in vitro or in vivo or as suture material. The cells to be grown can be mesenchymal stem cells, fully competent stem cells, expanded somatic lineages, separated and suspended cells, peripheral circulating cells and cells of either included or induced potential. In vivo cell growth can be for example achieved with respect to bone growth or with respect to healing processes of wounds. Suture material made of a product as explained above can also ameliorate healing processes at the suture sites since cells attach more easily to such a suture material than to conventional suture materials.

For example, the sheet material can be implanted into a body of a patient (either human or animal) and act as (temporary) bone substitute material. If it is made from a biodegradable or bioresorbable material like for example collagen, the sheet material will be resorbed or degraded over time and newly grown bone will replace the sheet material step by step. By promoting bone growth due to the specific structure of the sheet material, healing processes after operations are accelerated. The use of a sheet material as described above for enabling tissue growth either in vivo or in vitro is also part of the invention. Specifically, the use of a sheet material as described above as bone substitute material is encompassed by the invention. The sheet material may embody itself with charge that potentiates tissue differentiation. Stem cell differentiation has been closely tied to electrovolt membrane potential during phenotypic emergence. Electrospinning, charge potentiation, and multi-laminar sheet formation all carry the option to defined physical conditions of the matrix, and define voltage differences in regenerative tissues. Processed materials from electrospun biologic components, both linear and in piled matte, will be used to define matrix charge and enliven the differentiation process.

An embodiment of the disclosed invention will now be described making reference to an example.

In order to test the distribution and chemical stability of a product which was obtained by a method according to the preceding explanations, collagen as carrier material was electrospun together with NDGA as crosslinker. The obtained product was white, whereas practically all other applications making use of NDGA as crosslinker result in an orange or sepia toned material.

8-mm punches of the electrospun material were taken and placed in water, a solution of sodium hydroxide or a solution of acetic acid. Base oxidation immediately turned the material orange. The color change occurred uniformly.

The fact that the color change was uniform demonstrates that the crosslinker was uniformly distributed in the produced material. The fact that a base oxidation was still possible demonstrates that the material was not fully polymerized but is still polymerizable to a certain extent in a post-spinning process.

## Claims

1. Method for producing a nanofiber-based product, comprising the following steps:
- providing a carrier material solution comprising a carrier material,
- bringing the carrier material in contact with a collector by electrospinning the carrier material solution out of a spinning device, the collector having a first electrical polarity and the spinning device having a second electrical polarity being opposite to the first polarity,
**characterized**
**in that** the carrier material essentially consists of a polymer being, at least after having contacted the collector, embedded in a polymer formed by the crosslinker, the crosslinker being a compound according to the general formula (I) 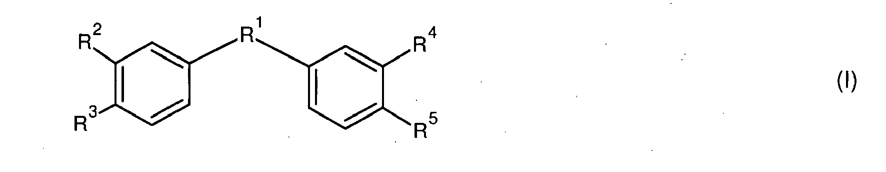 wherein R¹ is
- a single bond between the adjacent carbon atoms, or
- a carbohydrate chain having 1 to 10 carbon atoms and optionally bearing a hydroxy group, the carbohydrate chain being saturated, unsaturated or polyunsaturated,
and wherein R², R³, R⁴ and R⁵ are independently from each other
- a hydrogen,
- a carbohydrate chain having 1 to 10 carbon atoms and optionally bearing a hydroxy group, the carbohydrate chain being saturated, unsaturated or polyunsaturated,
- a hydroxy group, or
- a sulfhydryl group,
with the provision that the compound bears at least
- two hydroxy groups, or
- two sulfhydryl groups, or
- one hydroxy group and one sulfhydryl group.

2. Method according to claim 1, **characterized in that** R¹ is a residue having the following formula (II) 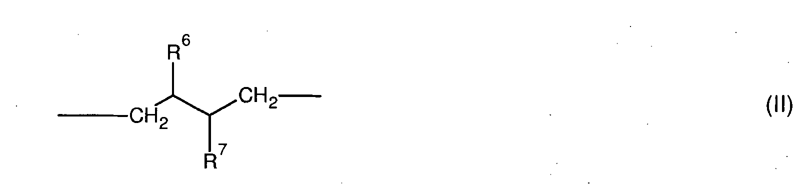 wherein each of the two indicated terminal methylene residues is linked to a carbon atom of each benzene ring via the single bond indicated in formula (II) and wherein R⁶ and R⁷ are independently from each other
- a hydrogen,
- a carbohydrate chain having 1 to 10 carbon atoms and optionally bearing a hydroxy group, the carbohydrate chain being saturated, unsaturated or polyunsaturated,
- a hydroxy group, or
- a sulfhydryl group.

3. Method according to claim 1 or 2, **characterized in that** at least two hydroxy groups or at least two sulfhydryl groups or at least one hydroxy group and one sulfhydryl group of the compound according to general formula (I) are bound to the benzene rings of this compound.

4. Method according to any of the preceding claims, **characterized in that** the crosslinker is nordihydroguaiaretic acid.

5. Method according to any of the preceding claims, **characterized in that** the crosslinker is used in an amount of 2 to 20 percent by mass with respect to the dry mass of the polymer of the carrier material.

6. Method according to any of the preceding claims, **characterized in that** the carrier material is crosslinked before it reaches the collector.

7. Method according to any of the preceding claims, **characterized in that** the carrier material comprises collagen, a mixture of collagen and hydroxy apatite, gelatin, alginates, chitosan, silk, cellulose, polyurethane, a polyester, polycaprolactone, polylactide, polypyrrole, polyaniline, polyacetylene, polythiophene, a copolymer of the preceding polymers and/or a copolymer bearing carboxylic acid groups and/or amine groups, as well as oligopeptides or polypeptides.

8. Method according to any of the preceding claims, **characterized in that** the electrospinning is done at a voltage of 8 to 20 kV between the collector and the spinning device.

9. Method according to any of the preceding claims, **characterized in that** the polymer is solved or dispersed in at least one liquid chosen from the group of water, alcohols like methanol or ethanol, aqueous solutions of acids or bases like acetic acid or sodium hydroxide and organic solvents like acetone or 1,1,1,3,3,3-hexafluoro-2-propanol to produce a carrier material solution.

10. Method according to any of the preceding claims, **characterized in that** the carrier material is deposited onto the collector to form a sheet material.

11. Method according to any of claims 1 to 9, **characterized in that** the carrier material is removed from the collector after having contacted it and is then spun to a yarn.

12. Product, in particular obtainable by a method according to any of the preceding claims, comprising a carrier material being built up from nanofibers having a diameter of less than 1200 nm and being
**characterized**
**in that** the carrier material essentially consists of a polymer being embedded in a polymer formed by the crosslinker, the crosslinker being a compound according to the general formula (I) 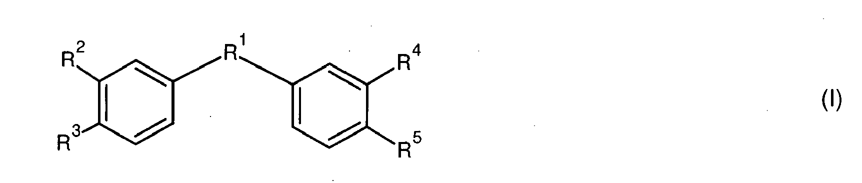 wherein R¹ is
- a single bond between the adjacent carbon atoms, or
- a carbohydrate chain having 1 to 10 carbon atoms and optionally bearing a hydroxy group, the carbohydrate chain being saturated, unsaturated or polyunsaturated,
and wherein R², R³, R⁴ and R⁵ are independently from each other
- a hydrogen,
- a carbohydrate chain having 1 to 10 carbon atoms and optionally bearing a hydroxy group, the carbohydrate chain being saturated, unsaturated or polyunsaturated,
- a hydroxy group, or
- a sulfhydryl group,
with the provision that the compound bears at least
- two hydroxy groups, or
- two sulfhydryl groups, or
- one hydroxy group and one sulfhydryl group.

13. Product according to claim 12, **characterized in that** the molecular ratio between each monomeric unit of the carrier material and each monomeric unit of the crosslinker is in the range of 20:1 to 5:1 in the product.

14. Product according to claim 12 or 13, **characterized in that** it further comprises at least one auxiliary substance of the group consisting of osteoinductive substances, electrically conductive substances, electrically semiconductive substances, electrically insulating substances, antibacterial substances, antiviral substances, antifungal substances, ceramics, barium, copper, bromine, niobium, lithium, germanium, titanium, lead, zirconium, silicon, silver, zinc, polyurethane and silver hydrogen sulfate, gallium orthophosphate, langasite, barium titanate, lead titanate, lead zirconate titanate, potassium niobate, lithium niobate, lithium tantalate, sodium tungstate, Ba₂NaNb₅O₅ and Pb₂KNb₅O₁₅, wherein the auxiliary substance is chemically and/or physically bound to the carrier material.

15. Use of a product according to any of claims 12 to 14 as scaffold for growing cells in vitro or as suture material.

## Patentansprüche

1. Verfahren zur Herstellung eines nanofaserbasierten Erzeugnisses, umfassend die folgenden Schritte:
- Bereitstellung einer ein Trägermaterial enthaltenden Trägermateriallösung,
- Inkontaktbringung des Trägermaterials mit einem Kollektor durch Elektrospinnen der Trägermateriallösung aus einer Spinnvorrichtung, wobei der Kollektor eine erste elektrische Polarität aufweist und die Spinnvorrichtung eine der ersten Polarität entgegengesetzte zweite elektrische Polarität aufweist,
**dadurch gekennzeichnet,**
**dass** das Trägermaterial im Wesentlichen aus einem zumindest nach Inkontaktbringung mit dem Kollektor in einem durch den Vernetzer gebildeten Polymer eingebetteten Polymer besteht, wobei es sich bei dem Vernetzer um eine Verbindung entsprechend der allgemeinen Formel (I) handelt: 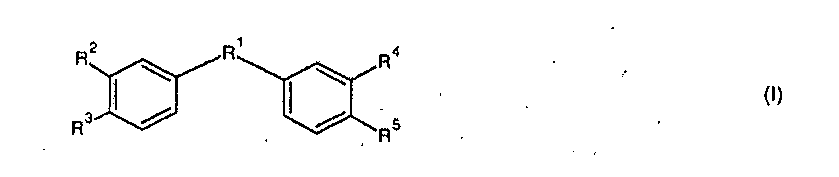 wobei R¹ für
- eine Einfachbindung zwischen den benachbarten Kohlenstoffatomen oder für
- eine gegebenenfalls eine Hydroxygruppe tragende Kohlenwasserstoffkette mit 1 bis 10 Kohlenstoffatomen steht, wobei die Kohlenwasserstoffkette gesättigt, ungesättigt oder mehrfach ungesättigt ist,
und wobei R², R³, R⁴ und R⁵ jeweils unabhängig voneinander für
- ein Wasserstoffatom,
- eine gegebenenfalls eine Hydroxygruppe tragende Kohlenwasserstoffkette mit 1 bis 10 Kohlenstoffatomen, wobei die Kohlenwasserstoffkette gesättigt, ungesättigt oder mehrfach ungesättigt ist,
- eine Hydroxygruppe oder
- eine Sulfhydrylgruppe stehen,
mit der Maßgabe, dass die Verbindung wenigstens
- zwei Hydroxygruppen oder
- zwei Sulfhydrylgruppen oder
- eine Hydroxygruppe und eine Sulfhydrylgruppe
trägt.

2. Verfahren nach Anspruch 1, **dadurch gekennzeichnet, dass** R¹ für einen Rest entsprechend der folgenden Formel (II) 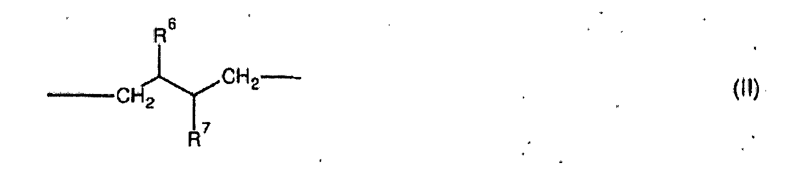 steht, wobei die zwei angegebenen endständigen Methylenreste jeweils über die in Formel (II) angegebene Einfachbindung mit einem Kohlenstoffatom jedes Benzolrings verknüpft sind und wobei R⁶ und R⁷ unabhängig voneinander für
- ein Wasserstoffatom,
- eine gegebenenfalls eine Hydroxygruppe tragende Kohlenwasserstoffkette mit 1 bis 10 Kohlenstoffatomen, wobei die Kohlenwasserstoffkette gesättigt, ungesättigt oder mehrfach ungesättigt ist,
- eine Hydroxygruppe oder
- eine Sulfhydrylgruppe stehen.

3. Verfahren nach Anspruch 1 oder 2, **dadurch gekennzeichnet, dass** wenigstens zwei Hydroxygruppen oder wenigstens zwei Sulfhydrylgruppen oder wenigstens eine Hydroxygruppe und eine Sulfhydrylgruppe der Verbindung entsprechend der allgemeinen Formel (I) mit den Benzolringen dieser Verbindung verbunden sind.

4. Verfahren nach einem der vorhergehenden Ansprüche, **dadurch gekennzeichnet, dass** es sich bei dem Vernetzer um Nordihydroguajaretsäure handelt.

5. Verfahren nach einem der vorhergehenden Ansprüche, **dadurch gekennzeichnet, dass** man den Vernetzer in einer auf die Trockenmasse des Polymers des Trägermaterials bezogenen Menge von 2 bis 20 Massenprozent einsetzt.

6. Verfahren nach einem der vorhergehenden Ansprüche, **dadurch gekennzeichnet, dass** das Trägermaterial vor Erreichen des Kollektors vernetzt wird.

7. Verfahren nach einem der vorhergehenden Ansprüche, **dadurch gekennzeichnet, dass** das Trägermaterial Collagen, eine Mischung von Collagen und Hydroxylapatit, Gelatine, Alginate, Chitosan, Seide, Cellulose, Polyurethan, einem Polyester, Polycaprolacton, Polylactid, Polypyrrol, Polyanilin, Polyacetylen, Polythiophen, ein Copolymer aus den vorhergehenden Polymeren und/oder ein Carbonsäuregruppen und/oder Amingruppen tragendes Copolymer sowie Oligopeptide oder Polypeptide umfasst.

8. Verfahren nach einem der vorhergehenden Ansprüche, **dadurch gekennzeichnet, dass** das Elektrospinnen bei einer Spannung zwischen dem Kollektor und der Spinnvorrichtung von 8 bis 20 kV erfolgt.

9. Verfahren nach einem der vorhergehenden Ansprüche, **dadurch gekennzeichnet, dass** zur Herstellung einer Trägermateriallösung das Polymer in mindestens einer Flüssigkeit ausgewählt aus der Gruppe bestehend aus Wasser, Alkoholen wie Methanol oder Ethanol, wässrigen Lösungen von Säuren oder Basen wie Essigsäure oder Natriumhydroxid und organischen Lösungsmitteln wie Aceton oder 1,1,1,3,3,3-Hexafluor-2-propanol gelöst oder dispergiert wird.

10. Verfahren nach einem der vorhergehenden Ansprüche, **dadurch gekennzeichnet, dass** das Trägermaterial auf dem Kollektor zur Bildung eines Flächenmaterials abgelegt wird.

11. Verfahren nach einem der Ansprüche 1 bis 9, **dadurch gekennzeichnet, dass** das Trägermaterial nach Inkontaktbringung mit dem Kollektor davon entfernt und anschließend zu einem Garn versponnen wird.

12. Erzeugnis, insbesondere erhältlich durch ein Verfahren gemäß einem der vorhergehenden Ansprüche, umfassend ein aus Nanofasern mit einem Durchmesser von weniger als 1200 nm aufgebautes Trägermaterial,
**dadurch gekennzeichnet,**
**dass** das Trägermaterial im Wesentlichen aus einem in einem durch den Vernetzer gebildeten Polymer eingebetteten Polymer besteht, wobei es sich bei dem Vernetzer um eine Verbindung entsprechend der allgemeinen Formel (I) handelt: 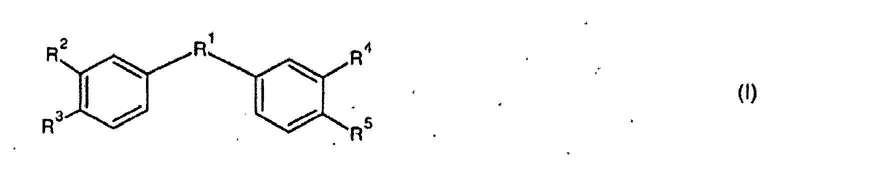 wobei R¹ für
- eine Einfachbindung zwischen den benachbarten Kohlenstoffatomen oder für
- eine gegebenenfalls eine Hydroxygruppe tragende Kohlenwasserstoffkette mit 1 bis 10 Kohlenstoffatomen steht, wobei die Kohlenwasserstoffkette gesättigt, ungesättigt oder mehrfach ungesättigt ist,
und wobei R², R³, R⁴ und R⁵ jeweils unabhängig voneinander für
- ein Wasserstoffatom,
- eine gegebenenfalls eine Hydroxygruppe tragende Kohlenwasserstoffkette mit 1 bis 10 Kohlenstoffatomen, wobei die Kohlenwasserstoffkette gesättigt, ungesättigt oder mehrfach ungesättigt ist,
- eine Hydroxygruppe oder
- eine Sulfhydrylgruppe stehen,
mit der Maßgabe, dass die Verbindung wenigstens
- zwei Hydroxygruppen oder
- zwei Sulfhydrylgruppen oder
- eine Hydroxygruppe und eine Sulfhydrylgruppe trägt.

13. Erzeugnis nach Anspruch 12, **dadurch gekennzeichnet, dass** im Erzeugnis das Molekularverhältnis zwischen jeder monomeren Einheit des Trägermaterials und jeder monomeren Einheit des Vernetzers im Bereich von 20 zu 1 bis 5 zu 1 liegt.

14. Erzeugnis nach Anspruch 12 oder 13, **dadurch gekennzeichnet, dass** es ferner mindestens einen Hilfsstoff aus der Gruppe bestehend aus osteoinduktiven Stoffen, elektrisch leitfähigen Stoffen, elektrisch halbleitfähigen Stoffen, elektrisch isolierenden Stoffen, antibakteriellen Stoffen, antiviralen Stoffen, antifungalen Stoffen, Keramik, Barium, Kupfer, Brom, Niobium, Lithium, Germanium, Titan, Blei, Zirkonium, Silizium, Silber, Zink, Polyurethan und Silberhydrogensulfat, Galliumorthophosphat, Langasit, Bariumtitanat, Bleititanat, Bleizirkonattitanat, Kaliumniobat, Lithiumniobat, Lithiumtantalat, Natriumwolframat, Ba₂NaNb₅O₅ und Pb₂KNb₅O₁₅ umfasst, wobei der Hilfsstoff chemisch und/oder physikalisch mit dem Trägermaterial verbunden ist.

15. Verwendung eines Erzeugnisses gemäß einem der Ansprüche 12 bis 14 als Gerüst für die in vitro-Aufzucht von Zellen oder als chirurgisches Nahtmaterial.

## Revendications

1. Procédé pour produire un produit à base de nanofibres, comprenant les étapes suivantes :
- fourniture d'une solution de matériau de support comprenant un matériau de support,
- mise en contact du matériau de support avec un collecteur par électrofilature de la solution de matériau de support à partir d'un dispositif de filature, le collecteur ayant une première polarité électrique et le dispositif de filature ayant une deuxième polarité électrique étant opposée à la première polarité,
**caractérisé**
**en ce que** le matériau de support est essentiellement constitué d'un polymère étant, au moins après avoir été mis en contact avec le collecteur, incorporé dans un polymère formé par l'agent de réticulation, l'agent de réticulation étant un composé selon la formule générale (I) 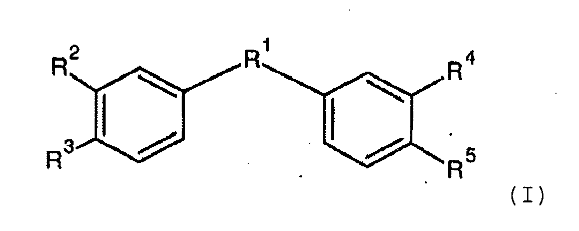 dans lequel R¹ est
- une simple liaison entre les atomes de carbone adjacents, ou
- une chaîne d'hydrocarbure ayant 1 à 10 atomes de carbone et comportant facultativement un groupe hydroxy, la chaîne d'hydrocarbure étant saturée, insaturée ou poly-insaturée,
et dans lequel R², R³, R⁴ et R⁵ sont indépendamment les uns des autres
- un hydrogène,
- une chaîne d'hydrocarbure ayant 1 à 10 atomes de carbone et comportant facultativement un groupe hydroxy, la chaîne d'hydrocarbure étant saturée, insaturée ou poly-insaturée,
- un groupe hydroxy, ou
- un groupe sulfhydryle,
à condition que le composé comporte au moins
- deux groupes hydroxy, ou
- deux groupes sulfhydryle, ou
- un groupe hydroxy et un groupe sulfhydryle.

2. Procédé selon la revendication 1, **caractérisé en ce que** R¹ est un résidu ayant la formule suivante (II) 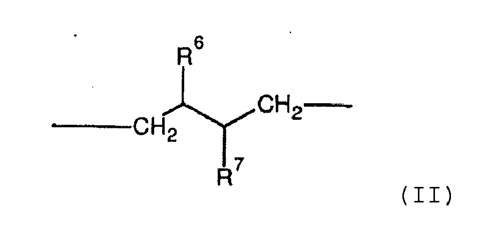 dans lequel chacun des deux résidus méthylène terminaux indiqués est lié à un atome de carbone de chaque cycle benzénique via la simple liaison indiquée dans la formule (II) et dans lequel R⁶ et R⁷ sont indépendamment l'un de l'autre
- un hydrogène,
- une chaîne d'hydrocarbure ayant 1 à 10 atomes de carbone et comportant facultativement un groupe hydroxy, la chaîne d'hydrocarbure étant saturée, insaturée ou poly-insaturée,
- un groupe hydroxy, ou
- un groupe sulfhydryle.

3. Procédé selon la revendication 1 ou 2, **caractérisé en ce qu'**au moins deux groupes hydroxy ou au moins deux groupes sulfhydryle ou au moins un groupe hydroxy et un groupe sulfhydryle du composé selon la formule générale (I) sont liés aux cycles benzéniques de ce composé.

4. Procédé selon l'une quelconque des revendications précédentes, **caractérisé en ce que** l'agent de réticulation est l'acide nordihydroguaiarétique.

5. Procédé selon l'une quelconque des revendications précédentes, **caractérisé en ce que** l'agent de réticulation est utilisé en une quantité de 2 à 20 pour cent en masse par rapport à la masse sèche du polymère du matériau de support.

6. Procédé selon l'une quelconque des revendications précédentes, **caractérisé en ce que** le matériau de support est réticulé avant d'atteindre le collecteur.

7. Procédé selon l'une quelconque des revendications précédentes, **caractérisé en ce que** le matériau de support comprend du collagène, un mélange de collagène et d'hydroxyapatite, de la gélatine, des alginates, du chitosane, de la soie, de la cellulose, un polyuréthane, un polyester, une polycaprolactone, un polylactide, un polypyrrole, une polyaniline, un polyacétylène, un polythiophène, un copolymère des polymères précédents et/ou un copolymère comportant des groupes acide carboxylique et/ou des groupes amine, ainsi que des oligopeptides ou des polypeptides.

8. Procédé selon l'une quelconque des revendications précédentes, **caractérisé en ce que** l'électrofilature est effectuée à une tension de 8 à 20 kV entre le collecteur et le dispositif de filature.

9. Procédé selon l'une quelconque des revendications précédentes, **caractérisé en ce que** le polymère est dissous ou dispersé dans au moins un liquide choisi dans le groupe de l'eau, d'alcools tels que le méthanol ou l'éthanol, de solutions aqueuses d'acides ou de bases tels que l'acide acétique ou l'hydroxyde de sodium et de solvants organiques tels que l'acétone ou le 1,1,1,3,3,3-hexafluoro-2-propanol pour produire une solution de matériau de support.

10. Procédé selon l'une quelconque des revendications précédentes, **caractérisé en ce que** le matériau de support est déposé sur le collecteur pour former un matériau en couche.

11. Procédé selon l'une quelconque des revendications 1 à 9, **caractérisé en ce que** le matériau de support est retiré du collecteur après avoir été mis en contact avec celui-ci et est ensuite filé en un fil.

12. Produit, en particulier pouvant être obtenu par un procédé selon l'une quelconque des revendications précédentes, comprenant un matériau de support étant constitué de nanofibres ayant un diamètre inférieur à 1200 nm et étant
**caractérisé**
**en ce que** le matériau de support est essentiellement constitué d'un polymère étant incorporé dans un polymère formé par l'agent de réticulation, l'agent de réticulation étant un composé selon la formule générale (I) 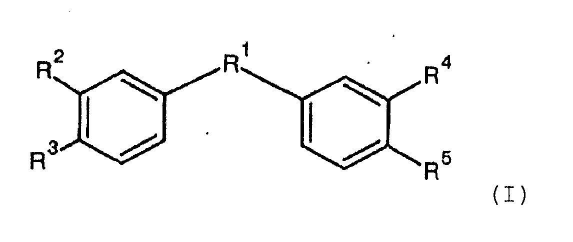 dans lequel R¹ est
- une simple liaison entre les atomes de carbone adjacents, ou
- une chaîne d'hydrocarbure ayant 1 à 10 atomes de carbone et comportant facultativement un groupe hydroxy, la chaîne d'hydrocarbure étant saturée, insaturée ou poly-insaturée,
et dans lequel R², R³, R⁴ et R⁵ sont indépendamment les uns des autres
- un hydrogène,
- une chaîne d'hydrocarbure ayant 1 à 10 atomes de carbone et comportant facultativement un groupe hydroxy, la chaîne d'hydrocarbure étant saturée, insaturée ou poly-insaturée,
- un groupe hydroxy, ou
- un groupe sulfhydryle,
à condition que le composé comporte au moins
- deux groupes hydroxy, ou
- deux groupes sulfhydryle, ou
- un groupe hydroxy et un groupe sulfhydryle.

13. Produit selon la revendication 12, **caractérisé en ce que** le rapport moléculaire entre chaque motif monomère du matériau de support et chaque motif monomère de l'agent de réticulation est dans la plage de 20:1 à 5:1 dans le produit.

14. Produit selon la revendication 12 ou 13, **caractérisé en ce qu'**il comprend en outre au moins une substance auxiliaire du groupe constitué de substances ostéo-inductrices, substances électriquement conductrices, substances électriquement semiconductrices, substances électriquement isolantes, substances antibactériennes, substances antivirales, substances antifongiques, céramiques, baryum, cuivre, brome, niobium, lithium, germanium, titane, plomb, zirconium, silicium, argent, zinc, polyuréthane et hydrogénosulfate d'argent, orthophosphate de gallium, langasite, titanate de baryum, titanate de plomb, zirconate-titanate de plomb, niobate de potassium, niobate de lithium, tantalate de lithium, tungstate de sodium, Ba₂NaNb₅O₅ et Pb₂KNb₅O₁₅, la substance auxiliaire étant chimiquement et/ou physiquement liée au matériau de support.

15. Utilisation d'un produit selon l'une quelconque des revendications 12 à 14 en tant qu'échafaudage pour cultiver des cellules *in vitro* ou en tant que matériau de suture.
